(19) Europäisches Patentamt
European Patent Office
Office européen des brevets

(11) **EP 3 901 954 A1**

(12) **EUROPEAN PATENT APPLICATION**

(43) Date of publication:
**27.10.2021 Bulletin 2021/43**

(51) Int Cl.:
**G16B 15/30** (2019.01)     **A61K 39/00** (2006.01)
**G16B 30/00** (2019.01)

(21) Application number: **20170484.8**

(22) Date of filing: **20.04.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **NEC OncoImmunity AS**
**0379 Oslo (NO)**

(72) Inventors:
• **SIMOVSKI, Boris**
**0379 Oslo (NO)**
• **Moliné, Clément**
**0379 Oslo (NO)**
• **STRATFORD, Richard**
**0379 Oslo (NO)**
• **CLANCY, Trevor**
**0379 Oslo (NO)**

(74) Representative: **Gill Jennings & Every LLP**
**The Broadgate Tower**
**20 Primrose Street**
**London EC2A 2ES (GB)**

Remarks:
Claims 16 to 28 are deemed to be abandoned due to non-payment of the claims fees (Rule 45(3) EPC).

(54) **METHOD AND SYSTEM FOR IDENTIFYING ONE OR MORE CANDIDATE REGIONS OF ONE OR MORE SOURCE PROTEINS THAT ARE PREDICTED TO INSTIGATE AN IMMUNOGENIC RESPONSE, AND METHOD FOR CREATING A VACCINE**

(57)     A computer-implemented method of identifying one or more candidate regions of one or more source proteins that are predicted to instigate an adaptive immunogenic response across a plurality of human leukocyte antigen, HLA, types, wherein the one or more source proteins has an amino acid sequence is disclosed. The method comprises (a) accessing the amino acid sequence of the one or more source proteins; (b) accessing a set of HLA types; (c) predicting an immunogenic potential of a plurality of candidate epitopes within the amino acid sequence, for each of the set of HLA types; (d) dividing the amino acid sequence into a plurality of amino acid sub-sequences; (e) for each of the plurality of amino acid sub-sequences, generating a region metric that is indicative of a predicted ability of the amino acid sub-sequence to instigate an immunogenic response across the set of HLA types, wherein the region metrics are based on the predicted immunogenic potentials of the plurality of candidate epitopes, for each of the set of HLA types; and (f) applying a statistical model to identify whether any of the generated region metrics are statistically significant, whereby an amino acid sub-sequence identified as having a statistically significant region metric corresponds to a candidate region of the amino acid sequence that is predicted to instigate an immunogenic response across at least a subset of the set of HLA types. A corresponding system is also disclosed, as well as a method for creating a vaccine.

Fig. 7

S201 — Access amino acid sequence of one more source proteins

S203 — Identify a plurality of candidate epitopes within the amino acid sequence

S205 — Predict an immune response potential for each of the candidate epitopes, for each of a set of HLA types

S207 — Assign an epitope score to each amino acid, for each HLA type, based on the overlapping candidate epitope having the best predicted immunogenic potential for the HLA type

S208 — Digitise epitope scores

S209 — Divide the amino acid sequence into a plurality of amino acid sub-sequnces

S211 — For each amino acid sub-sequence, calculate a region metric based on the assigned epitope scores

S213 — Apply a statistical model to identify candidate regions having statistically significant region metrics

S215 — Filter candidate regions occurring in conserved regions

**Description**

**INTRODUCTION**

Well established as an effective form of epidemiological control, vaccines have had significant success in aiding the decline of infections and mortalities associated with viral infections such as smallpox and polio. Other infections, however, for example those caused by Coronaviridae such as Severe Acute Respiratory Syndrome Coronavirus (SARS-CoV), SARS-CoV-2 and Middle East Respiratory Syndrome Coronavirus (MERS-CoV), have proven harder to vaccinate against.

Much of the global efforts to develop a Coronaviridae vaccine to date have focused primarily on stimulating an antibody response against the exposed spike glycoprotein (S-protein), serving as the most exposed structural protein on the virus. However, although responses against the S-protein of SARS-CoV have been shown to confer short-term protection in mice (Yang et al. 2004, Nature 428(6982): 561-4), neutralising antibody responses against the same structure in convalescent patients are typically of low titre and short-lived (Channappanavar et al. 2014, Immunol Res 88(19): 11034-44) (Yang et al. 2006, Clin Immunol 120(2) 171-8). Furthermore, the induction of antibody responses to S-protein in SARS-CoV has been associated with harmful effects in some animal models, raising possible safety concerns. In macaque models, for example, it was observed that anti-S-protein antibodies were associated with severe acute lung injury (Liu et al. 2019 JCI Insight 4(4)), whilst sera from SARS-CoV patients also revealed that elevated anti-S-protein antibodies were observed in those patients that succumbed to the disease.

Further concerns over an S-protein-centred approach arise when considering the possibility of antibody-dependent enhancement (ADE), a biological phenomenon wherein antibodies facilitate viral entry into host cells and enhance the infectivity of the virus (Tirado & Yoon 2003, Viral Immunol 16(1) 69-86). It has been demonstrated that a neutralising antibody may bind to the S-protein of a Coronavirus, triggering a conformational change that facilitates viral entry (Wan et al. J Virol 2020, 94(5)).

Due to these problems, it is therefore desirable to develop additional strategies for vaccine design, such as the use of T cell antigens designed to instigate a broad T cell immune response in the recipient.

However, when considering vaccines designed to instigate a broad T cell response, there exists a further challenge of human leukocyte antigen (HLA) restriction within an individual and a broader population. An HLA system is a gene complex encoding the major histocompatibility complex (MHC) proteins in humans, responsible for the regulation of an individual's immune system, as well as the ability to specifically present epitopes at the surface of an infected cell, and elicit an immune response against epitopes from intracellular pathogens, and epitopes delivered to said individual in the form of a vaccine (Marsh et al. 2010 Tissue Antigens 75(4): 291-455).

The high polymorphism of HLA alleles and subsequent immune system variability between individuals results in a diverse spectrum of "HLA types" across the population. As an added complication, such HLA types can have a significant impact on the efficacy of a potentially prophylactic viral vaccine composition between different individuals. As such, the design and generation of an epitope-based vaccine that is compatible with a particular subset of HLA types may prove ineffective with a significant proportion of the global population comprising individuals of different HLA types.

Therefore, there is a need to develop methods for designing and creating vaccines with the potential to stimulate a broad adaptive immune response across a significant proportion of the global population.

**SUMMARY OF THE INVENTION**

According to a first aspect of the invention, there is provided a computer-implemented method of identifying one or more candidate regions of one or more source proteins that are predicted to instigate an adaptive immunogenic response across a plurality of human leukocyte antigen, HLA, types, wherein the one or more source proteins has an amino acid sequence, the method comprising: (a) accessing the amino acid sequence of the one or more source proteins; (b) accessing a set of HLA types; (c) predicting an immunogenic potential of a plurality of candidate epitopes within the amino acid sequence, for each of the set of HLA types; (d) dividing the amino acid sequence into a plurality of amino acid sub-sequences; (e) for each of the plurality of amino acid sub-sequences, generating a region metric that is indicative of a predicted ability of the amino acid sub-sequence to instigate an immunogenic response across the set of HLA types, wherein the region metrics are based on the predicted immunogenic potentials of the plurality of candidate epitopes, for each of the set of HLA types; and (f) applying a statistical model to identify whether any of the generated region metrics are statistically significant, whereby an amino acid sub-sequence identified as having a statistically significant region metric corresponds to a candidate region of the amino acid sequence that is predicted to instigate an immunogenic response across at least a subset of the set of HLA types.

The method of the present invention advantageously uses a statistical model to quantitatively analyse the predicted immunogenic potential of one or more candidate epitopes - in other words the predicted ability of the one or more candidate epitopes to instigate an immunogenic response - within an amino acid sub-sequence, across a set of different HLA types. The candidate regions (or "hotspots") of the amino acid sequence that are identified by the quantitative statistical analysis may represent regions (e.g. areas) of the one or more source proteins that are most likely to be viable vaccine targets and may be used in vaccine design and creation. In particular, the identified candidate regions are likely to contain one or more viable T-cell epitopes ("predicted epitopes") that may instigate a broad T-cell immune response across a population having therein a set of different HLA types.

The term "epitope" as used herein refers to any part of an antigen that is recognised by any antibodies, B cells, or T cells. An "antigen" refers to a molecule capable of being bound by an antibody, B cell or T cell, and may be comprised of one or more epitopes. As such, the terms epitope and antigen may be used interchangeably herein. Epitopes may also be referred to by the molecule for which they bind, such as "T cell epitopes", or more specifically, "MHC Class I epitopes" or "MHC Class II epitopes".

The human leukocyte antigen (HLA) system is a complex of genes encoding the MHC proteins in humans. Owing to the highly polymorphic nature of HLA genes, in which the term "polymorphic" refers to a high variability of different alleles, the precise MHC proteins of each human individual coded by varying HLA genes may differ to fine-tune the adaptive immune system. Many hundreds of different alleles have been recognised for HLA molecules. The terms HLA type and HLA allele may be used interchangeably herein.

The region metric for an amino acid sub-sequence is indicative of the predicted immunogenic potential of the one or more candidate epitopes within the amino acid sub-sequence, across the tested set of HLA types. Thus, a "relatively better" region metric indicates that the one or more candidate epitopes within that amino acid sub-sequence are collectively predicted to instigate an immunogenic response across a large proportion of the HLA types. A "relatively worse" region metric indicates that the one or more candidate epitopes within that amino acid sub-sequence are not collectively predicted to instigate an immunogenic response across a large proportion of the HLA types in the analysis.

The statistical model is applied to identify those amino acid sub-sequences having a statistically significant region metric. In particular, the statistical model is applied to identify any region metric that is better than expected by chance. As would be understood by the skilled person, the significance threshold of the statistical modelling may be chosen accordingly, for example based on the perceived accuracy of the predicted immunogenic potential of the candidate epitopes.

A candidate region may comprise a single candidate epitope that is predicted to instigate an immunogenic response across a plurality of the HLA types (a "viable" or "predicted" epitope). Such an epitope may be termed as "overlapping with" a number of HLA types. More typically however, a candidate region comprises a plurality of candidate epitopes that are predicted to instigate an immunogenic response and that, collectively, overlap with a large proportion of the analysed HLA types. For example, one viable epitope within a candidate region may overlap with $n$ HLA types and a different viable epitope within the candidate region may overlap with m HLA types such that the candidate region is predicted to instigate an immunogenic response across the ($m+n$) HLA types.

It is envisaged that the predicted epitopes may differ in length from each other, and may overlap with each other. For example, a candidate region may comprise a predicted epitope of 8 amino acids in length, in addition to a further predicted epitope of 25 amino acids in length, wherein said predicted epitope of 25 amino acids in length may overlap with part of, or fully comprise the entirety of, the predicted epitope of 8 amino acids in length.

Typically, the method may further comprise the step of assigning, for each of the set of HLA types, an epitope score to each amino acid, wherein the epitope score is based on the predicted immunogenic potentials of one or more of the candidate epitopes comprising that amino acid, for that HLA type; and wherein each of the region metrics is generated based on the epitope scores for the amino acids within the respective amino acid sub-sequence, across the set of HLA types.

Thus, by generating the region metrics based on the epitope scores for the amino acids within the respective amino acid sub-sequence (which are in turn indicative of the immunogenic potential of a corresponding candidate epitope), each region metric is indicative of the ability of the amino acid sub-sequence to instigate an immunogenic response across the set of HLA types.

The region metric may be an average of the amino acid epitope scores within the respective amino acid sub-sequence, across the set of HLA types.

In embodiments, at least a subset of the epitope scores may be assigned by: (i) identifying a first plurality of candidate epitopes having a first (typically fixed) length, across the amino acid sequence; (ii) generating, for each of the set of HLA types, an epitope score for each of the first plurality of candidate epitopes that is indicative of the predicted immunogenic potential of the respective candidate epitope for that HLA type; (iii) identifying a second plurality of candidate epitopes having a second (typically fixed) length, across the amino acid sequence; (iv) generating, for each of the set of HLA types, an epitope score for each of the second plurality of candidate epitopes that is indicative of the predicted immunogenic potential of the respective candidate epitope for that HLA type; and (v) for each of the set of HLA types, assigning, for each amino acid of the amino acid sequence, the epitope score of the candidate epitope that is predicted to have the best immunogenic potential of all of the first and second candidate epitopes comprising that amino acid, for that HLA type.

The first plurality of candidate epitopes are firstly identified across the amino acid sequence, preferably in a "moving window" of amino acids of fixed length. In such a "moving window" approach, the step size between consecutive candidate epitopes is less than the length of the candidate epitopes, such that the consecutive candidate epitopes overlap. Typically, the step size is one amino acid. This is performed for each HLA type. For each of the candidate epitopes of the first plurality, an epitope score is generated that is indicative of the immunogenic potential of that candidate epitope, for the respective HLA type. We will consider how these epitope scores are generated in more detail later.

A second plurality of candidate epitopes are subsequently identified across the amino acid sequence, for each HLA type. Again, this is preferably performed using a "moving window approach". Each of the second epitopes is also assigned an epitope score that is indicative of the immunogenic potential of that epitope, for the respective HLA type.

Each amino acid is then assigned, for each HLA type, the epitope score of the candidate epitope that is predicted to have the best immunogenic potential of all the candidate epitopes comprising that amino acid. Hence, for a particular HLA type, if candidate epitope "A" and candidate epitope "B" both comprised a particular amino acid "X", the amino acid "X" would be assigned the epitope score of whichever candidate epitope "A" or "B" is predicted to have the best immunogenic potential. In other words, for a given HLA type, the epitope score allocated to an amino acid corresponds to the best score obtained by a candidate epitope overlapping with this amino acid.

The candidate epitopes of the first plurality and the candidate epitopes of the second plurality have different lengths.

The method typically extends to identifying a third, and more, plurality of candidate epitopes in the same manner. For example, when considering Class I HLA types, candidate epitope of lengths of 8, 9, 10, 11 and 12 amino acids may be identified and scored based on the associated predicted immunogenic potential. Thus, in embodiments, a plurality of 8-mer candidate epitopes across the amino acid sequence may be identified and scored, then a plurality of 9-mers, a plurality of 10-mers, a plurality of 11-mers and 12-mers identified and scored. Each amino acid may then be allocated the epitope score corresponding to the best score obtained by one of the identified candidate epitopes that comprises that amino acid.

Preferably, the candidate epitopes have a length of at least 8 amino acids, preferably wherein the candidate epitopes have a length of 8, 9, 10, 11, 12 or 15 amino acids. Typically, candidate epitopes of length between 8 and 12 amino acids are identified for Class I HLA types, and candidate epitopes of length 15 amino acids are identified for class II HLA types, although other lengths may be used.

In preferred embodiments, the predicted immunogenic potential of a candidate epitope for a particular HLA type is based on one or more of: a predicted binding affinity and a predicted processing of the identified candidate epitope.

Preferably, the predicted immunogenic potential (or "immunogenicity") of a candidate epitope is based on both a predicted binding affinity and processing of the candidate epitope. The combination of the predicted binding affinity and a predicted processing may be termed a predicted presentation of the candidate epitope. However, good results may still be obtained if the predicted immunogenic potential is based one of these metrics (e.g. for Class II HLA types, good results have been obtained when the candidate epitopes are predicted for percentile rank binding affinity scores).

Such predictions may be performed using an antigen presentation or binding affinity prediction algorithm, experimental data, or both. Examples of publically available databases and tools that may be used for such predictions include the Immune Epitope Database (IEDB) (https://www.iedb.org/), the NetMHC prediction tool (http://www.cbs.dtu.dk/services/NetMHC/), the TepiTool prediction tool (http://tools.iedb.org/tepitool/), the MHCflurry prediction tool, the NetChop prediction tool (http://www.cbs.dtu.dk/services/NetChop/) and the MHC-NP prediction tool (http://tools.immuneepitope.org/mhcnp/.). Other techniques are disclosed in WO2020/070307 and WO2017/186959.

In particularly preferred embodiments, antigen presentation is predicted from a machine learning model that integrates in an ensemble machine learning layer information from several HLA binding predictors (e.g. trained on ic50nm binding affinity data) and a plurality of different predictors of antigen processing (e.g. trained on mass spectrometry data).

The immunogenic potential may be based on alternative means of measuring the foreignness or ability to stimulate an immune response of a candidate epitope. Such examples might include comparing the candidate epitopes to determine how similar they are is to a pathogen database, or prediction models that attempt to learn the physicochemical differences between immunogenic epitopes non-immunogenic peptides.

In embodiments, immunogenic potential of a candidate epitope may be further based on a similarity of the candidate epitope to a human protein. Thus, candidate epitopes may be penalised (e.g. assigned a lower score) if they are similar to a human protein.

An advantageous feature of the present invention is that the method not only identifies candidate regions comprising epitopes that may bind to a HLA molecule, but also those CD8 epitopes that are naturally processed by a cell's antigen processing machinery, and presented on the surface of the host infected cells.

The method may further comprise digitising ("binarising") the assigned epitope scores, wherein each epitope score meeting a predetermined criterion is transformed to a "1" and each epitope score not meeting the predetermined criterion is transformed to a "0". The region metric for an amino acid sub-sequence may then typically be calculated as an average, across the set of HLA types, number of amino acids within the sub-sequence with the value "1" assigned.

After the digitising process, amino acids assigned an epitope score of "1" may be considered as comprising part of a viable epitope predicted to instigate an immunogenic response. Thus, regions of amino acids having an assigned score of "1" may contain one or more (possibly overlapping) candidate epitopes predicted to bind multiple HLA types.

Preferably, the set of HLA types includes HLA types of Major Histocompatibility Complex, MHC, Class I and HLA types of MHC Class II. In this way, the method is advantageously capable of predicting candidate regions predicted to instigate a broad T cell response across CD8+ and CD4+ T cell types. However, useful results may be obtained if the set of HLA types includes only HLA types of MHC Class I or only HLA types of MHC Class II.

The set of HLA types may comprise HLA types representative of exactly one human population group. A population group may be an ethnic population group (e.g. Caucasian, Africa, Asian) or a geographical population group (e.g. Lombardy, Wuhan). Thus, the invention may be used to identify candidate regions for a particular population group. Identified candidate regions that are common for a number of different population groups are thus particularly advantageous for use in creating a vaccine.

In embodiments, the set of HLA types may comprise HLA types representative of different human population groups. In this way, the method of the present invention may beneficially be used to identify candidate regions that are predicted to provide an immunogenic response across a large proportion of the human population.

In preferred embodiments, the set of HLA types comprises HLA types representative of the human population. In this way, candidate regions that are predicted to instigate an immunogenic response over a majority (or all) of the HLA types within such a set of HLA types may be viable candidates for a "universal" vaccine.

The set of HLA types may comprise the top N most frequent HLA types within the human population or human population group, preferably wherein N is at least 5, more preferably at least 50 and even more preferably wherein N=100. The statistical model of the present invention is particularly advantageous as it allows candidate regions to be identified for a large number (e.g. 100) of HLA types. In this way, the present invention may be used to design and create vaccines with the potential to stimulate a broad adaptive immune response across a significant proportion of the global population.

Although the present invention has particular benefit for identifying candidate regions predicted to provide an immunogenic response across a large proportion of the human population, it may also be used to generated personalised vaccines for an individual (e.g. for cancer therapeutic vaccines in the neoantigen field). Thus, in embodiments, the set of HLA types may be representative of a given individual.

It will be appreciated that different candidate regions may be identified by the method of the present invention, based on the set of HLA types used.

The statistical model may in general be based on one or more parametric distributions (e.g. binomial, Poisson or hypergeometric distributions) or sampling methods in order to identify statistically significant amino acid sub-sequences. In particularly preferred embodiments, applying the statistical model comprises applying a Monte Carlo simulation to estimate a p-value for each of the generated region metrics. The estimated p-values are then used to identify the statistically significant amino acid sub-sequences and, consequently, the candidate regions. The use of a Monte Carlo algorithm is particularly advantageous as it allows the complexities in producing the epitope scores to be reflected in the null model.

The null model for statistical modelling is typically defined as the generative model of the set of epitope scores, for each HLA type, if they were to be generated by chance. The set of epitope scores for a particular HLA type may be referred to as an "HLA track". The Monte Carlo simulation may be used to iteratively produce a set of randomised HLA tracks and a plurality of associated simulated region metrics, from which the p-value - and hence the statistical significance - of a region metric may be estimated.

It is preferable that the null model reflects the complexities behind the generation of the epitope scores. Thus, preferably, applying the Monte Carlo simulation includes: (i) for each HLA type, arranging the epitope scores into a plurality of epitope segments and epitope gaps based on the distribution of the epitope scores; and (ii) for each HLA type, iteratively generating a random arrangement of the epitope segments and epitope gaps.

The arrangement of the epitope scores for each HLA type (arrangement of each HLA track) into a plurality of epitope segments and epitope gaps reflects whether the amino acid was part of a candidate epitope predicted to have a good immunogenic potential or not, based on its assigned score. Thus, an epitope segment is a consecutive sequence of (typically at least 8) epitope scores assigned to amino acids within an epitope predicted to have a good immunogenic potential. Such an epitope segment made up of a sequence of "epitope amino acids" may be considered as an amino acid region containing one or more predicted epitopes that may or may not overlap with each other. An epitope gap is one or more consecutive scores assigned to amino acids that are not part of such predicted epitopes. By iteratively randomising the epitope segments and epitope gaps rather than individual amino acid epitope scores, the null model more faithfully reflects the methodology behind the region metrics, thereby providing a more reliable result.

The method may further comprise applying a false discovery rate, FDR, procedure to the results of the statistical model, preferably wherein the FDR procedure is the Benjamin-Hochberg procedure or Benjamini-Yekutieli procedure.

In embodiments, the epitope scores may be weighted dependent upon the human population frequency of the respective HLA type within the set of HLA types. Thus, candidate epitopes that are predicted to instigate an immunogenic response across the most frequent HLA types may be given preferential weighting which is reflected in the epitope scores of the amino acids. Statistically significant amino acid sub-sequences are identified as candidate regions that are likely to be viable vaccine targets. Thus, the size of the amino acid sub-sequences are typically chosen based on the intended vaccine platform. Preferably, each amino acid sub-sequence has the same length. For example, in step (b) of the method the amino acid sequence may be divided into a plurality of amino acid sub-sequences of length between 20 and 50 amino acids for peptide vaccine platforms where identified candidate region(s) may be synthesised. Longer amino acid sub-sequences (e.g. of between 50 and 150 amino acids) may be used for vaccine platforms based on encoding the candidate region(s) into a corresponding DNA or RNA sequence. It is also envisaged that protein domains identified to have a large T-cell epitope population may be used in vaccines. Such domains may provide a conformational antibody response.

Particularly preferred amino acid sub-sequence sizes are 27 amino acids, 50 amino acids or 100 amino acids.

Although the amino acid sub-sequences are typically chosen to have the same length, they may be chosen to have different lengths. The amino acid sub-sequences may overlap with each other such that they span the amino acid sub-sequence in a "moving window" approach as discussed above. However, in order to reduce computational resources required to run the statistical model, the amino acid sub-sequences may be chosen not to overlap, e.g. they may be arranged in a contiguous manner across the amino acid sequence.

The candidate regions identified in the method as explained so far are predicted to contain viable T-cell epitopes that may instigate a broad T-cell immune response across a population having therein a set of different HLA types. In preferred embodiments, each of the region metrics may be further indicative of a predicted B-cell response potential of the respective amino acid sub-sequence. In other words, the region metric may be indicative of the presence of any B-cell epitopes within the amino acid sub-sequence. In some embodiments, each assigned epitope score may be further based on the predicted B cell response potential of the respective amino acid (e.g. within a predicted B-cell epitope). Additionally or alternatively, the method may further comprise analysing each candidate region of the one or more source proteins for the presence of B cell epitopes.

B-cell response predictions may be based on B-cell binding prediction algorithms, experimental data, or both. One example of a prediction tool that may be used in such embodiments is the BepiPred prediction tool (http://www.cbs.dtu.dk/services/BepiPred/).

In embodiments, the method may further comprise comparing each identified candidate region with at least one human protein sequence in order to determine a degree of similarity, and ranking, filtering or discarding the candidate regions based on the degree of similarity with at least one of the human proteins being greater than a predetermined threshold.

These techniques advantageously compares the similarity of the identified candidate regions with the expression profile of proteins expressed in different key organs in order to avoid adverse responses to vaccines based on such candidate regions. Different predetermined thresholds may be used. For example, a candidate region may be discarded if it contains one or more epitopes exactly matching a human protein.

The method may comprise adjusting a candidate region based on one or more adjacent amino acid sub-sequences. For example, if a candidate region is identified but it is known that the adjacent amino acid sub-sequence has a predicted T cell epitope close to the border between the two sub-sequences, the amino acid sequence of the candidate region may be extended to include the further epitope. It will also be appreciated that identified candidate regions may be combined together. For example, two 50 amino acid candidate regions may be combined to form a 100 amino acid candidate region for use in a vaccine. The one or more source proteins are preferably one or more proteins of a virus, bacterium, parasite or tumour, or fragments thereof. The one or more source proteins may include neoantigens. For example, the one or more source proteins may be one or more of the Spike (S) protein, Nucleoprotein (N), Membrane (M) protein, Envelope (E) protein, as well as open reading frames such as ORF10, ORF1AB, ORF3A, ORF6, ORF7A, ORF8. Thus, the method of the present invention may be applied to an entire viral proteome. This is particularly beneficial for the identification of candidate regions for vaccine design. In embodiments, the source protein may be one or more proteins of a coronavirus, preferably the SARS-CoV-2 virus.

The one or more source proteins may be or comprise a plurality of variations of one or more source proteins, (and/or the method may be applied to a plurality of variations of the one or more source proteins). Each variation may be a mutation of a virus protein for example. In this way, the method of the present invention may advantageously be used to analyse the immunogenicity of all of the non-synonymous variations across a plurality of different protein sequences (e.g. of a virus). The method may advantageously comprise filtering the one or more candidate regions so as to select one or more candidate regions in conserved areas of the one or more proteins (i.e. areas less likely to present mutations). Conserved regions may be identified using techniques known in the art.

The amino acid sequence of the one or more source proteins may be obtained by one of: oligonucleotide hybridisation methods, nucleic acid amplification based methods (including but not limited to polymerase chain reaction based methods), automated prediction based on DNA or RNA sequencing, de novo peptide sequencing, Edman sequencing or mass spectrometry. The amino acid sequence may be downloaded from a bioinformatic depository such as UniProt (www.uniprot.org).

The method may further comprise synthesising one or more identified candidate regions, and/or one or more predicted ("viable") epitopes within the one or more identified candidate regions.

The method may further comprise encoding the one or more identified candidate regions, and/or one or more predicted ("viable") epitopes within the one or more identified regions, into a corresponding DNA or RNA sequence. Such DNA or RNA sequences may be incorporated into a delivery system for use in a vaccine (e.g. using naked or encapsulated DNA, or encapsulated RNA). The method may comprise incorporating the DNA or RNA sequence into a genome of a bacterial or viral delivery system to create a vaccine.

Thus, according to a second aspect of the invention there is provided a method of creating a vaccine, comprising: identifying at least one candidate region of at least one source protein by any of the methods of the first aspect disclosed above; and synthesising the at least one candidate region and/or at least one predicted epitope within the at least one candidate region, or encoding the at least one candidate region and/or at least one predicted epitope within the at least one candidate region into a corresponding DNA or RNA sequence. Such a DNA or RNA sequence may be delivered in a naked or encapsulated form, or incorporated into a genome of a bacterial or viral delivery system to create a vaccine. In addition, bacterial vectors can be used to deliver the DNA in to vaccinated host cells. For peptide vaccines, the candidate region(s) and/or epitope(s) may typically be synthesised as an amino acid sequence or "string".

In accordance with a third aspect of the invention there is provided a system for identifying one or more candidate regions of one or more source proteins that are predicted to instigate an immunogenic response across a plurality of human leukocyte, HLA allele types, wherein the one or more source proteins has an amino acid sequence, the system comprising at least one processor in communication with at least one memory device, the at least one memory device having stored thereon instructions for causing the at least one processor to perform any of the methods of the first aspect disclosed above.

In accordance with a fourth aspect of the invention there is provided a computer readable medium having computer executable instructions stored thereon for implementing the any of the methods of the first aspect disclosed above.

## BRIEF DESCRIPTION OF THE DRAWINGS

Embodiments will now be described in detail, by way of example only, with reference to the accompanying figures, in which:

Figures 1A and 1B illustrate epitope maps of the S-protein of the SARS-CoV-2 virus across the most frequent HLA-A, HLA-B and HLA-DRB alleles in the human population. In these epitope maps the data has been transformed such that a positive result for CD8 relates to 0.7 or above, and 10% (represented by 0.1 in the figure) or below for Class II. Broad coverage for CD8 and CD4 is demonstrated with overlaying B cell antibody support;

Figure 2 shows hierarchical clustering of binary transformation of the epitope maps for Class I CD8 epitopes in HLA-A and HLA-B alleles for the S-protein of the SARS-CoV-2 virus;

Figure 3 illustrates epitope hotspots from a Monte Carlo analysis captured across the entire viral proteome of the SARS-CoV-2 virus using filtering procedures for conserved and human self-peptides;

Figure 4 is a scatter plot showing the mutated AP score against its wildtype AP score protein variant;

Figure 5 illustrates application of a Monte Carlo epitope hotspot prediction to 10 mutating virus sequences in different geographical locations;

Figure 6 illustrates scatter plots showing the distribution of hotspot conservation scores for proteins in a viral genome;

Figure 7 is a flow diagram showing the steps of a preferred embodiment of the method;

Figure 8 is an example of a system suitable for implementing embodiments of the method is shown; and

Figure 9 is an example of a suitable server.

## DETAILED DESCRIPTION OF THE DRAWINGS

According to certain embodiments described herein there is proposed a method and system for identifying one or more candidate regions of one or more source proteins that are predicted to instigate an adaptive immunogenic response across a plurality of HLA types. Such candidate regions may be referred to as "hotspots", and the terms "candidate region" and "hotspots" may be used interchangeably herein. In embodiments, the identified hotspots and/or epitopes identified therein may be used in vaccine design and creation.

We now describe a preferred embodiment for identifying such hotspots may be identified. Although the following description is in reference to an analysis of the entire proteome of the SAR-Cov-2 virus, it will be understood that the present invention may be utilised for an analysis of different viruses, tumours, bacteria or parasites, or fragments thereof such as neoantigens.

Generation of global epitope maps and amino acid scores

For a given HLA allele, the score allocated to an amino acid corresponds to the best score obtained by an epitope prediction overlapping with this amino acid. For Class I HLA alleles, the epitope lengths are preferably 8, 9, 10 and 11 and 12, and predicted for antigen presentation (AP) or immune presentation (IP) of the viral peptide to host-infected cell surface. Various methods and tools may be used to predict for AP, for example publically available NETCHop and NETMHC prediction tools, as well as those discussed in the summary section herein. These Class I scores range between 0 and 1, where by 1 is the best score (i.e., higher likelihood of being naturally presented on the cell surface). In this embodiment, for class II HLA alleles, we have made predictions on is 15mers. The Class II were predictions were percentile rank binding affinity scores (not antigen presentation), so the lower scores are best (the scores range from 0 to 100, with 0 being the best score).

Statistical framework for the detection of epitope hotspot epitope regions in different HLA populations

*Input data*

The data sets inputted into the statistical framework are epitope maps generated for each amino-acid position in the one or more source proteins (e.g. all the proteins in the SARS-CoV-2 proteome), for all of the studied (e.g. 100 HLA alleles). A score for any given amino acid was determined as the maximum AP or IP score that a peptide (candidate epitope) overlapping that amino acid holds in the epitope map. All peptide lengths of size 8-11 amino acids for class I, and 15 for class II were processed, generating one HLA dataset per viral protein. Each row in the dataset represents the amino acid epitope scores predicted for one HLA type.

*Statistical framework*

The central question that the statistical framework attempts to answer is: "are specific regions in a given viral protein enriched with higher immunogenic scores, with respect to a given set of HLA types, more than expected by chance?"

*HLA tracks*

The raw input datasets (e.g. the AP or percentile rank binding affinity scores) are first transformed into binary tracks. For each class I HLA dataset, the epitope scores are transformed to binary (0 and 1) values, such that amino-acid positions with predicted epitope scores larger than 0.7 (for AP) and larger than 0.5 (for IP) are assigned the value 1 (positively predicted epitope), and the rest are assigned the value 0. Similarly, for class II HLA datasets, amino-acid positions with predicted epitope scores smaller than 10 are assigned the value 1, otherwise 0. These thresholds were relatively conservative, and it will be appreciated that other thresholds may be chosen based on the techniques and confidence in the generation of the raw data. Each binary track can effectively be presented as a list of intervals of consecutive ones - segments, with consecutive zeros in between, forming inter-segments or gaps.

*Test statistic*

For a group of *k* HLA binary tracks, a test statistic ("region metric") Si is calculated for each bin *bi* of given size m, dividing the protein in n bins (e.g. m=100 amino-acids for the larger proteins). For a single HLA track, a test statistic $s_i$ is calculated for each bin $b_i$

$$s_i = \sum_{j=1}^{m} b_{i,j} * weight_k$$

where the weight is by default 1, however can also represent frequency of the HLA track in the population under analysis. Then, for *i=1..n,*

$$S_i = \frac{\sum_{i=1}^{k} s_i}{k}$$

which is the average number of amino-acids predicted to be epitopes (epitope enrichment) of the bin *bi,* across the selected HLA types.

*Null model*

An effective approach to estimate the statistical significance of the observed HLA tracks is Monte Carlo-based simulations. A null model is defined, as the generative model of the HLA tracks, if they were generated by chance. From the null model, through sampling, arises the null distribution of the test statistic Si. The null model must reflect the complexities behind the nature of the HLA tracks. Epitope amino acids in one HLA track will always form consecutive groups of length at least 8 (smallest peptide size used in the prediction framework). Similarly, amino acids with low epitope scores will also cluster together.

*P-value estimation*

To sample from the null model, each of the k HLA tracks is divided in segments and gaps, which are then shuffled to produce a randomized HLA track. In this embodiment, this is repeated 10000 times, to produce 10000 samples of *Si* statistic for each bin. For each bin, the p-value is estimated as the proportion of the samples that are equal or larger then the truly observed enrichment. Further, the generated p-values are adjusted for multiple testing with the Benjamini-Yekutieli procedure to control for a false discovery rate (FDR) of 0.05, although it will be appreciated that other multiple testing procedures (e.g. Benjamini Hochberg) may be used. Different false discovery rates may be implemented.

**Epitope hotspot conservation scores**

An example of generating a measure of conservation is now described. For each protein within the viral genome, the set of unique amino acid sequences was compiled from all the strains available in the GISAID database (Shu, Y. and J. McCauley, GISAID: Global initiative on sharing all influenza data - from vision to reality. Euro Surveill, 2017. 22(13)) as of 29.03.2020. These sets were individually processed using the Clustal Omega (v1.2.4) (Sievers, F. and D.G. Higgins, Clustal Omega for making accurate alignments of many protein sequences. Protein Sci, 2018. 27(1): p. 135-145.) software via the command line interface with default parameter settings. The software outputs a consensus sequence that contains conservation information for each amino acid within the protein sequence. As such, an amino acid depicted as an "*" at position i within the consensus sequence translates to that amino acid being conserved at position i among all the input sequences (Sievers, F. and D.G. Higgins, Clustal Omega for making accurate alignments of many protein sequences. Protein Sci, 2018. 27(1): p. 135-145.)

The hotspot offsets were then used to extract their respective consensus sub-sequence. For each hotspot, the conservation score was calculated as the ratio of "*" within its consensus sub-sequence to the total length of the sub-sequence. Accordingly, each hotspot was assigned a conservation score between 0 and 1, with 1 representing a perfect conservation across all available strains.

The median conservation score was calculated by sampling 1,000 sub-sequences equal to the hotspot size from the entire consensus sequence of a protein. Each sample was assigned a conservation score and the median value from all 1,000 conservation scores was calculated. The minimum conservation score was calculated using a sliding window approach, with the window size being equal to the hotspot size. For each increment, a conservation score was calculated and the resulting minimum conservation score was kept.

We now describe an example of applying the method of the present invention to the SARS-CoV-2 virus proteome. However, as has been discussed above, the method may be applied to a number of different source proteins such as different viruses, bacteria, tumours or parasites. The method may be applied to neoantigens.

The immunogenic landscape of SARS-CoV-2 reveals diversity among the different HLA groups in the human population

We carried out an epitope mapping of the entire SARS-CoV-2 virus proteome. Antigen presentation (AP) was predicted from a machine-learning model that integrates in an ensemble machine learning layer information from several HLA binding predictors (in the case three distinct HLA binding predictors trained on ic50nm binding affinity data) and 13 different predictors of antigen processing (all trained on mass spectrometry data). The outputted AP score ranges from 0 to 1, and was used as input to compute immune presentation (IP) across the epitope map. The IP score penalizes those presented peptides that have degrees of "similarity to human" when compared against the human proteome, and awards peptides that are less similar. The resulting IP score represents those HLA presented peptides that are likely to be recognized by circulating T-cells in the periphery i.e. T-cells that have not been deleted or anergized, and therefore most likely to be immunogenic.

Both the AP and the IP epitope predictions are "pan" HLA or HLA -agnostic and can be carried out for any allele in the human population, however for the purpose of this study we limited the analysis to 100 of the most frequent HLA-A, HLA-B and HLA-DR alleles in the human population. Class II HLA binding predictions were also incorporated into the large scale epitope screen from the IEDB consensus of tools (Dhanda, S.K., et al., IEDB-AR: immune epitope database-analysis resource in 2019. Nucleic Acids Res, 2019. 47(W1): p. W502-W506.), and B cell epitope predictions were performed using BepiPred (Dhanda, S.K., et al., IEDB-AR: immune epitope database-analysis resource in 2019. Nucleic Acids Res, 2019. 47(W1): p. W502-W506.). The resulting epitope maps allowed for the identification of regions in the viral proteome that are most likely to be presented by host-infected cells using the most frequent HLA-A, HLA-B and HLA-DR alleles in the global human population.

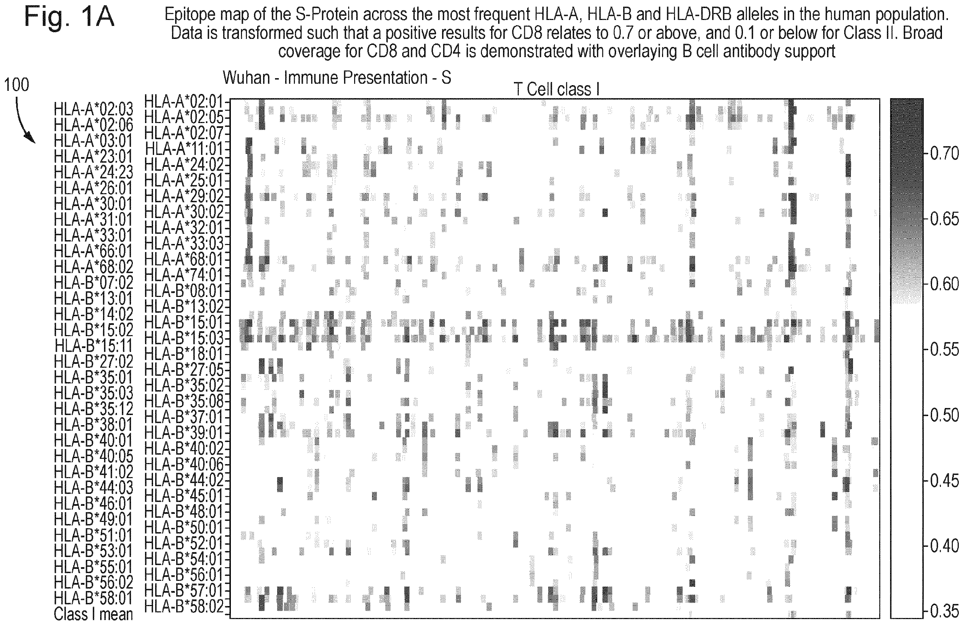

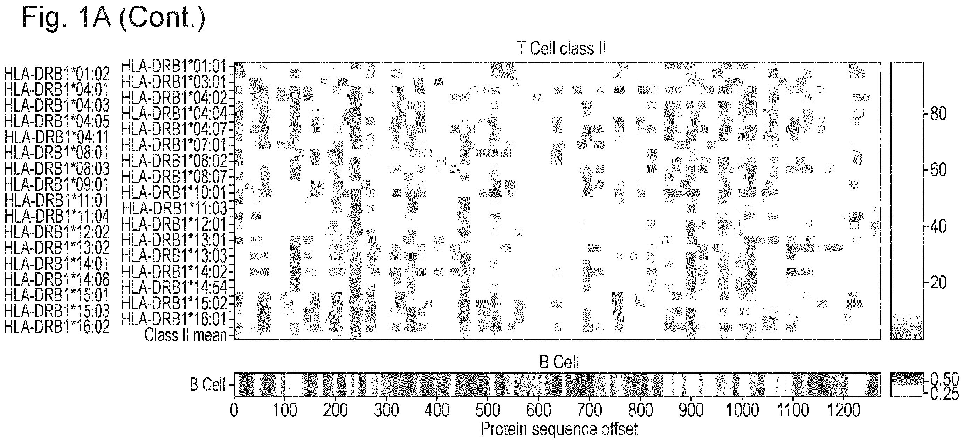

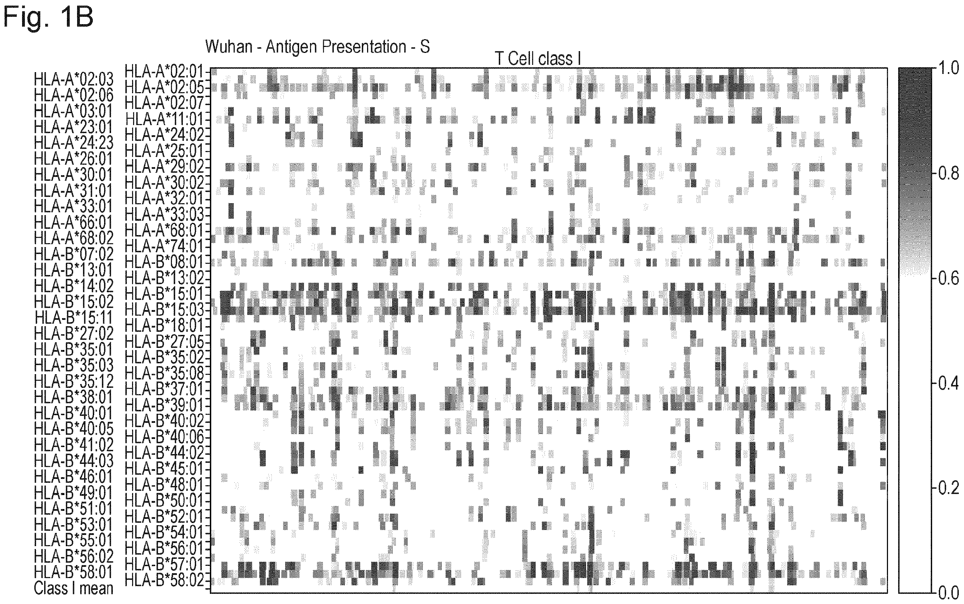

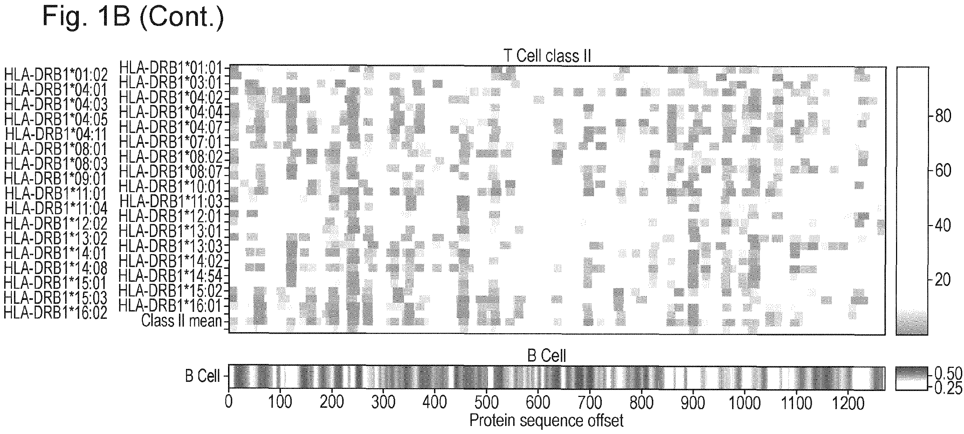

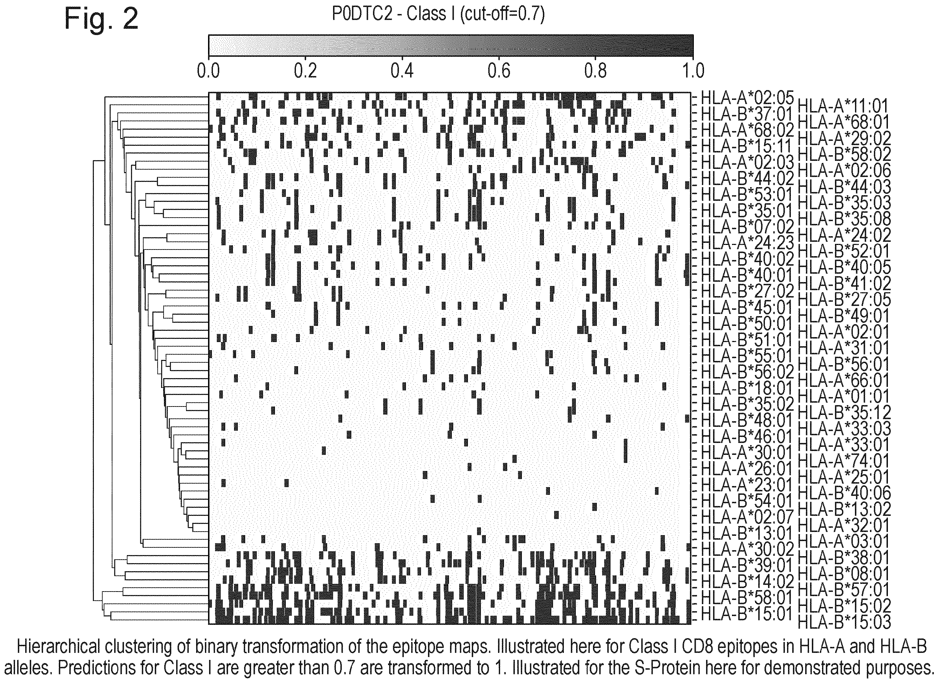

Epitope maps were created for all of the viral proteins and an example based on the IP scores for the S-protein is depicted in Figure 1A and for AP in Figure 1B, and illustrates distinct regions of the S-protein that contain candidate CD8 and CD4 epitopes for the 100 most frequent human HLA-A, HLA-B and HLA-DR alleles. This set of HLA types is indicated at 100 in Fig. 1A. Interestingly, the predicted B cell epitopes often map to regions of the protein that contain a high density of predicted T cell epitopes, thus the heat maps provide an overview of the most relevant regions of the SARS-CoV-2 virus that could be used to develop a vaccine. It is clear from Figure 1 that different HLA alleles have different Class I AP, and Class II binding properties. This strongly suggests, as one might anticipate, that the SARS-CoV-2 antigen presentation landscape clusters into distinct population groups across the spectrum of different human HLA alleles. This trend is further illustrated in the hierarchichal clustering maps presented Figure 2 after the AP scores have been binarized. Figure 2 clearly demonstrates that some allelic clusters present many viral targets to the human immune system, while others only present a few targets, and some are unable to present any. Figure 2 illustrates epitope segments and epitope gaps that may be shuffled, for each HLA type, in a Monte Carlo simulation. This implies that different groups in the human population with different HLA's will respond differentially to a T cell driven vaccine composed of viral peptides. Therefore in order to design the optimal vaccine that leverages the benefits of T cell immunity across a broad human population it is desirable to predict "epitope hotspots" in the viral proteome. These hotspots are regions of the virus that are enriched for overlapping epitopes, and or epitopes in close spatial proximity, that can be recognized by multiple HLA types across the human population.

Prior to discovery of such epitope hotspots that have the broadest coverage in the human population, we validated, to the extent that is possible from the limited number of validated SARS-CoV viral epitopes, that the T cell based AP and IP scores are predicting viable targets. We identified class I epitopes from the original SARS-CoV virus (that first emerged in the Guangdong province in China in 2002) that shared ≥90% sequence identity with the current SARS-CoV-2. Unfortunately, many of the published epitopes were identified using ELISPOT on PBMCs from convalescent patients and/or healthy donors (or humanised mouse models) where the restricting HLA was not explicitly deconvoluted. In order to circumvent this problem, we identified a subset of 5 epitopes where the minimal epitopes and HLA restriction had been identified using tetramers (Grifoni, A., et al., A Sequence Homology and Bioinformatic Approach Can Predict Candidate Targets for Immune Responses to SARS-CoV-2. Cell Host Microbe, 2020).

Four out of the 5 epitopes tested were identified as positive i.e. had an IP score of above 0.5 (see Table 1) demonstrating an accuracy of 80%. Although this was a very small test dataset, this provides us some degree of confidence that the *NEC Immune Profiler* prediction pipeline can accurately identify good immunogenic candidates and that the epitope hotspots identified by this analysis and subsequent analyses represent interesting targets for vaccine development.

**TABLE 1**

| Peptide | Sequence similarity | Parental protein | IP score |
|---|---|---|---|
| FIAGLIAIV | 100% | Spike glycoprotein | 0.54 |
| MEVTPSGTWL | 100% | Nucleoprotein | 0.61 |
| RLNEVAKNL | 100% | Spike glycoprotein | 0.39 |
| TLACFVLAAV | 100% | Membrane protein | 0.54 |
| KLPDDFTGCV | 90% | Spike glycoprotein | 0.58 |

A robust statistical analysis identifies epitope hotspots for a broad T cell response.

In order to identify epitope hotspots that have the potential to be viable immunogenic targets for the vast majority of the human population, we first carried out a Monte Carlo random sampling procedure, on the epitope maps generated previously (for the Wuhan reference sequence exemplified in Figure 1 for the S-protein), to identify specific areas of the SARS-CoV-2 proteome that have the highest probability of being epitope hotspots using the methods described above. Three bin sizes were investigated for potential epitope hotspots; 27, 50 and 100. A statistic was calculated for each defined subset region of the protein (bin) from the set of 100 HLAs. The Monte Carlo simulation method was then used to estimate the p-values for each bin, whereby each bin represented a candidate epitope hotspot. The statistically significant bins that emerged from the simulation represented epitope hotspot or regions of interest for each protein analyzed.

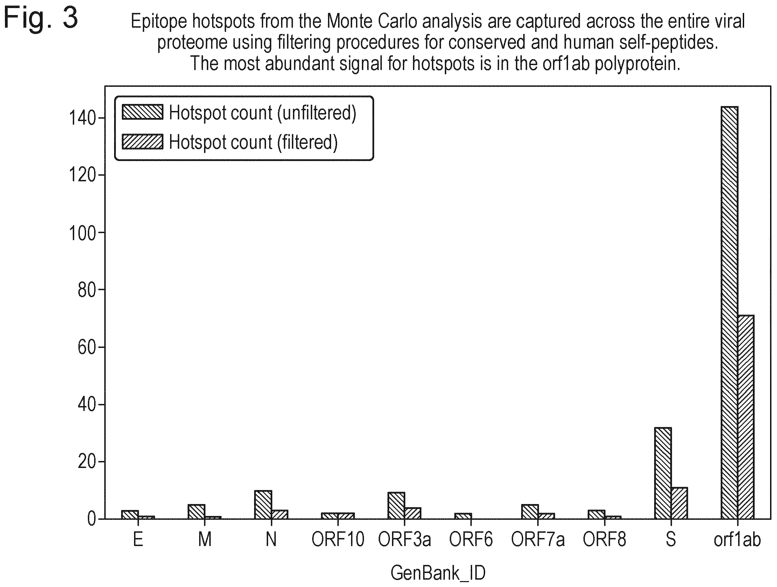

Epitope hotspots are built on the individual epitope scores, epitope lengths, and for each amino acid that they comprise. These scores are generated for each amino acid in the hotspots for all of the 100 HLA alleles most frequent in the human population. Based on the Monte Carlo analysis, the significant hotspots are those below a 5% false discovery rate (FDR), and represent regions that are most likely to contain viable T cell driven vaccine targets that can be recognized by multiple HLA types across the human population. A summary of the epitope hotspots identified across the entire spectrum of the virus is depicted in Figure 3 and reveals that the most immunogenic regions of the virus, that target the most frequent Human HLA alleles in the global population, are found in several of the viral proteins above and beyond the antibody exposed structural proteins, such as the S protein.

Conservation analysis identifies robust epitope hotspots in SARS-CoV-2

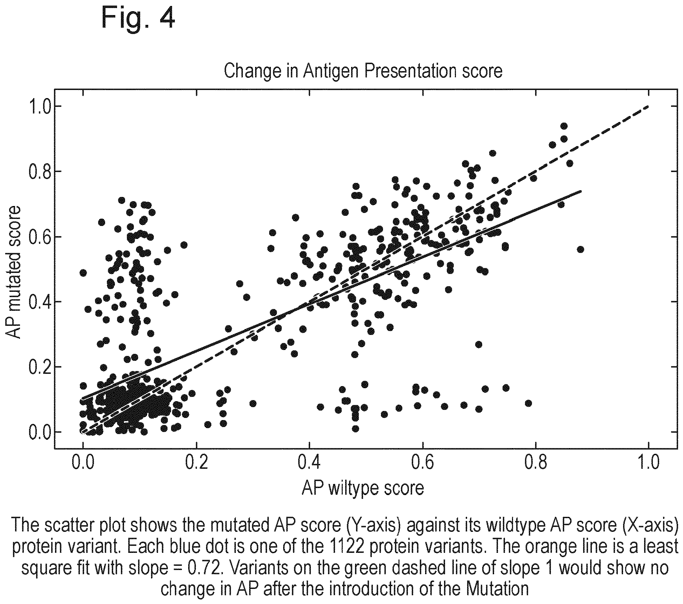

A universal vaccine blueprint should ideally also be able to protect populations against different emerging clades of the SARS-COV-2 virus and we therefore compared the AP potential of 3400 virus sequences in the GISAID database against the AP potential of the Wuhan Genbank reference sequence. The outcome of that comparison is illustrated in Figure 4, and hints at a trend whereby SARS-COV-2 mutations seem to reduce their potential to be presented and consequently detected by the host immune system. Similar trends have been observed in chronic infections such as HPV and HIV.

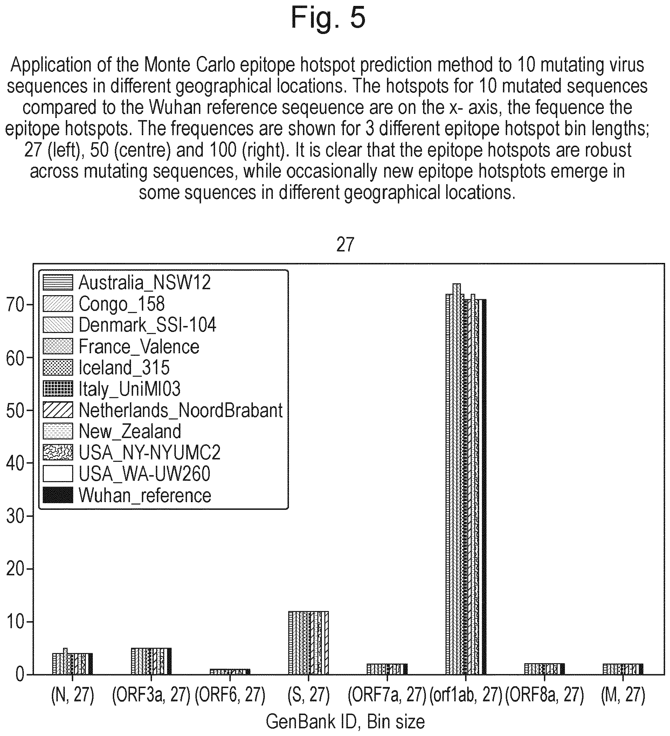

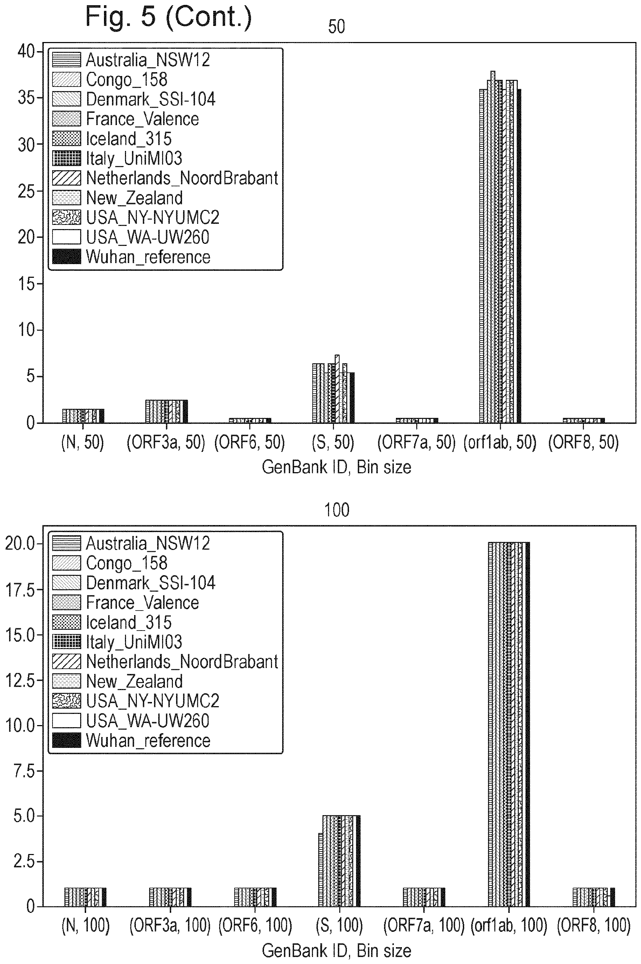

In order to assess if these epitope hotspots are sufficiently robust across all the sequenced and mutating strains of SARS-CoV-2, we next used the epitope hotspot Monte Carlo statistical framework, and analyzed 10 sequences of the virus from among the 10 most mutated viral sequences from different geographical regions (Shu, Y. and J. McCauley, GISAID: Global initiative on sharing all influenza data - from vision to reality. Euro Surveill, 2017. 22(13)). The vast majority of the hotspots were present in all of the sequenced viruses, however occasionally hotspots were eliminated and/or new hotspots emerged in these divergent strains. This is illustrated in Figure 5. Figure 5 illustrates application of the Monte Carlo epitope hotspot prediction method to 10 mutating virus sequences in different geographical locations. The hotspots for 10 mutated sequences compared to the Wuhan reference sequence are on the x-axis, the frequency of the epitope hotspots on the y axis. The frequencies are shown for three different hotspot bin lengths; 27 (left), 50 (centre) and 100 (right). It is clear that the epitope hotspots are robust across mutating sequences, while occasionally new epitope hotspots emerge in some sequences in different geographical locations.

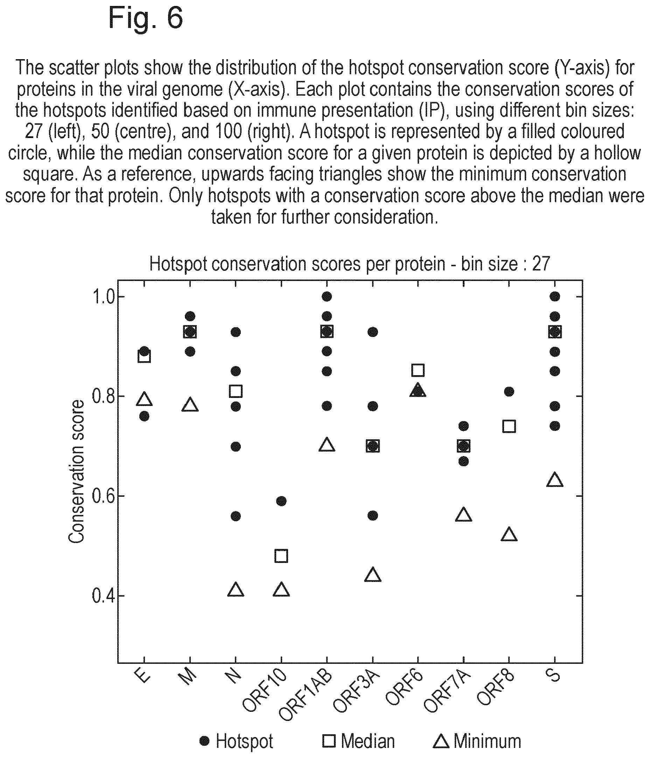

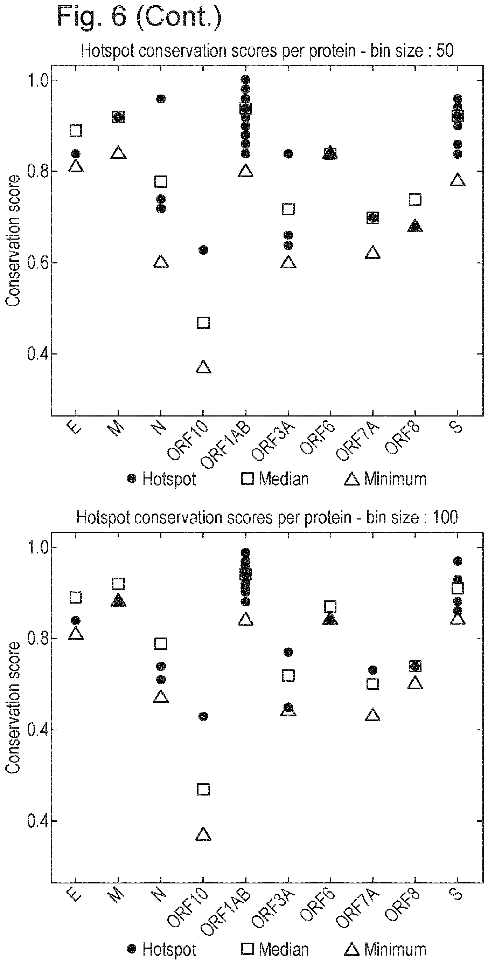

Although the identified hotspots seem to be robust across different viral strains, in order to design the most robust vaccine blueprint that will hopefully provide broad protection against new emerging clades of the SARS-COV-2 virus, the epitope hotspots were subject to a sequence conservation analysis. The goal of this analysis was to identify hotspots that appear to be less prone to mutation across thousands of viral sequences. We calculated a conservation score for each hotspot based on the consensus sequence of a protein using the techniques discussed above. Figure 6 shows conservation scores for the hotspots identified based on IP using different bin sizes. Only the epitope hotspots presenting a conservation score higher than the median conservation score were kept for further analysis. This allowed us to filter out approximately half of the hotspots for bin sizes of 50 and 100 amino acids and >70% for a bin size of 27 amino acids. In addition, to reduce the potential for off-target autoimmune responses against host tissue we removed bins that contained exact sequence matches to proteins in the human proteome.

Variant immunogenic potential across the mutating sequences of SARS-CoV-2

We downloaded all the strains available in the GISAID database (Shu, Y. and J. McCauley, GISAID: Global initiative on sharing all influenza data - from vision to reality. Euro Surveill, 2017. 22(13)) as of 31.03.2020, and ran them through the Nexstrain/Augur software suite with default parameters (Hadfield, J., et al., Nextstrain: real-time tracking of pathogen evolution. Bioinformatics, 2018. 34(23): p. 4121-4123). We parsed the resulting phylogenic tree to obtain all protein variants. For each we computed a wildtype score and a mutated Antigen Presentation (AP) score for HLA-A*02:01. The mutated score is the maximum AP score among the nine possible 9-mers peptides that include the variant.

The wildtype score is the maximum AP score for the 9-mers at the same positions in the reference (Wuhan) strain.

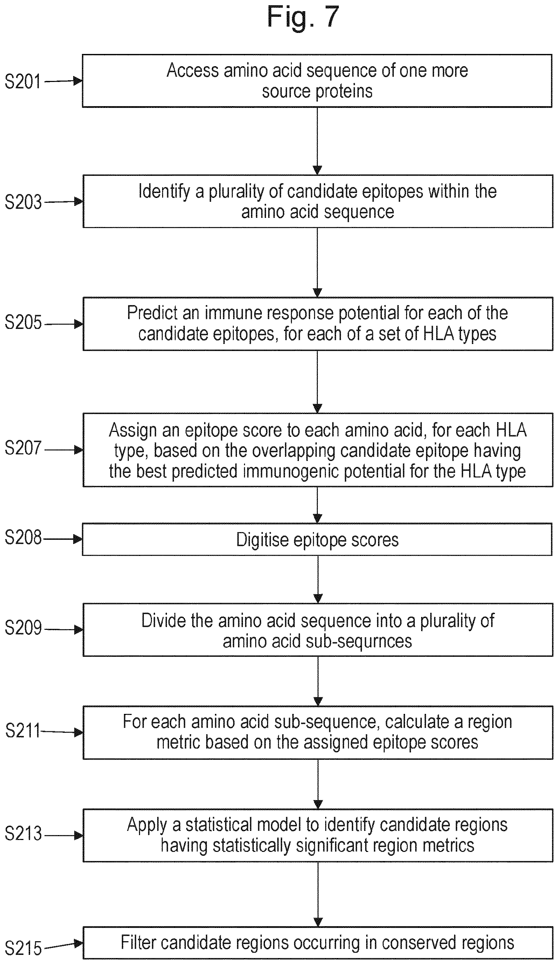

Figure 7 is a flow chart summarising the steps of a preferred embodiment of the present invention, which steps have been discussed in more detail above.

At step S201, an amino acid sequence of one or more source proteins is obtained. These may be one or more source proteins of a virus, bacteria, parasite or tumour, for example.

At step S203, a plurality of candidate epitopes are identified within the amino acid sequence. These candidate epitopes may have lengths of 8, 9, 10, 11, 12 or 15 amino acids and may be identified in a "moving window" approach, for example.

At step S205, an immune response potential is predicted for each candidate epitope, for each of a set of HLA types (e.g. representative of a human population). The immune response potential may be an antigen presentation (AP) or immune presentation (IP) score as discussed above.

At step S207, each amino acid, for each HLA type, is assigned an epitope score based on the overlapping candidate epitope having the best predicted immunogenic potential for the HLA type. The epitope score may be the AP or IP value for example.

At step S208, the epitope scores are digitised into epitope segments and epitope gaps, based on a predetermined threshold. Epitope segments are indicative of viable epitopes for an HLA type.

At step S209, the amino acid sequence is divided into a plurality of amino acid sub-sequences, or "bins". These may have varying length dependent on the intended vaccine platform, for example.

At step S211, a region metric is calculated for each amino acid sub-sequence, based on the assigned epitope scores within an amino acid sub-sequence.

At step S213, a statistical model (such as a Monte Carlo simulation) is used to identify candidate regions (or "hotspots") having a statistically significant region metric.

At Step S215, the identified candidate regions may be filtered to prioritise those that occur in conserved regions. For example, different sequences of a virus sequence may be analysed, and candidate regions identified in conserved regions across the different analyses may be prioritised.

In this document, we provide a clear use of the method in the design of vaccines. However, it will be understood that the techniques described herein could equally apply to designing T-cells that recognise epitope(s) in the identified candidate regions ("hotspots"). Similarly, the techniques could also be used to identify neoantigen burden in a tumour are where this is used as a biomarker, i.e. predicting response to a therapy.

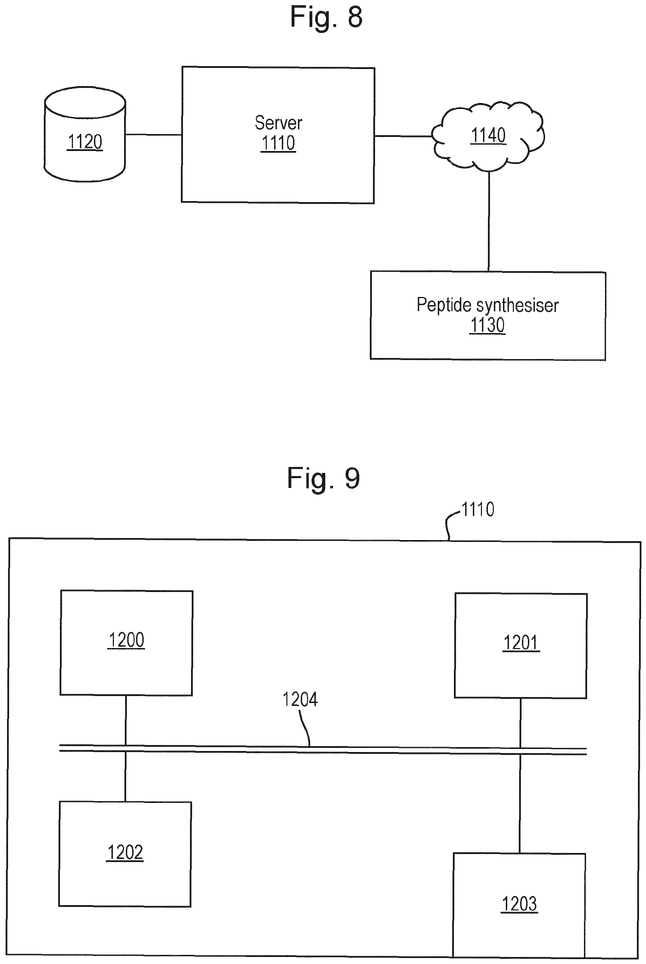

Turning now to Figure 8, an example of a system suitable for implementing embodiments of the method is shown. The system 1100 comprises at least one server 1110 which is in communication with a reference data store 1120. The server may also be in communication with an automated peptide synthesis device 1130, for example over a communications network 1140.

In certain embodiments the server may obtain, for example using from the reference data store, an amino acid sequence of one or more source proteins, together with data related to a set of HLA types. The server may then identify one or more candidate hotspots of the amino acid sequence using the steps described above.

The candidate regions (or one or more predicted epitopes within a candidate region) may be sent to the automated peptide synthesis device 1130 to synthesise the candidate region or epitopes. Such peptide synthesis is particularly pertinent for candidate regions or epitopes up to 30 amino acids in length. Techniques for automated peptide synthesis are well known in the art and it will be understood that any known technique may be used. Typically, the candidate region or epitope is synthesized using standard solid phase synthetic peptide chemistry and purified using reverse-phase high performance liquid chromatography before being formulated into an aqueous solution. If used for vaccination, prior to administration the peptide solution is usually admixed with an adjuvant before being administered to the patient

Peptide synthesis technology has existed for more than 20 years but has undergone rapid improvements in recent years to the point where synthesis now takes just a few minutes on commercial machines. For brevity we do not describe in detail such machines but their operation would be understood to one skilled in the art and such conventional machines may be adapted to receive a candidate region or epitope from the server.

The server may comprise the functions described above to identify candidate regions on an amino acid sequence. It will of course be understood that these functions may be subdivided across different processing entities of a computer network and different processing modules in communication with one another.

The techniques for identifying candidate regions may integrate into a wider ecosystem for customised vaccine development (e.g. using the method of the present invention for HLA types of an individual). Example vaccine development ecosystems are well known in the art and are described at a high-level for context, but for brevity we do not describe the ecosystem in detail.

In an example ecosystem, a first, sample, step may be to isolate DNA from a tumor biopsy and matched healthy tissue control. In a second, sequence, step, the data is sequenced and the variants identified i.e. the mutations. In an immune profiler step the associated mutated peptides may be generated «in silico».

Using the associated mutated peptides, and the techniques described here, a candidate region may be predicted and selected and target epitopes identified for vaccine design. That is, the candidate peptide sequence chosen based on its predicted binding affinity determined using the technique described herein.

The target epitopes are then generated synthetically using conventional techniques as described above. Prior to administration the peptide solution is usually admixed with an adjuvant before being administered to the patient (vaccination). In alternatives, the target epitopes can be engineered into DNA or RNA, or engineered into the genome of a bacteria or virus, as with any conventional vaccine.

The candidate regions predicted by the methods described herein may also be used to create other types of vaccine other than peptide based vaccines. For example the candidate regions (or predicted epitopes therein) could be encoded into the corresponding DNA or RNA sequence and used to vaccinate the patient. Note that the DNA is usually inserted in to a plasmid construct. Alternatively the DNA can be incorporated into the genome of a bacterial or viral delivery system (can be RNA also - depending on the viral delivery system) - which can be used to vaccinate the patient - so the manufactured vaccine in a genetically engineered virus or bacteria which manufactures the targets post immunisation in the patient i.e. in vivo.

An example of a suitable server 1110 is shown in Figure 9. In this example, the server includes at least one microprocessor 1200, a memory 1201, an optional input/output device 1202, such as a keyboard and/or display, and an external interface 1203, interconnected via a bus 1204 as shown. In this example the external interface 1203 can be utilised for connecting the server 1110 to peripheral devices, such as the communications networks 1140, reference data store 1120, other storage devices, or the like. Although a single external interface 1203 is shown, this is for the purpose of example only, and in practice multiple interfaces using various methods (e.g. Ethernet, serial, USB, wireless or the like) may be provided.

In use, the microprocessor 1200 executes instructions in the form of applications software stored in the memory 1201 to allow the required processes to be performed, including communicating with the reference data store 1120 in order to receive and process input data, and/or with a client device to receive sequence data for one or more source proteins, and to generate immunogenic potential predictions (e.g. including predicted binding affinity and processing) according to the methods described above. The applications software may include one or more software modules, and may be executed in a suitable execution environment, such as an operating system environment, or the like.

Accordingly, it will be appreciated that the server 1200 may be formed from any suitable processing system, such as a suitably programmed client device, PC, web server, network server, or the like. In one particular example, the server 1200 is a standard processing system such as an Intel Architecture based processing system, which executes software applications stored on non- volatile (e.g., hard disk) storage, although this is not essential. However, it will also be understood that the processing system could be any electronic processing device such as a microprocessor, microchip processor, logic gate configuration, firmware optionally associated with implementing logic such as an FPGA (Field Programmable Gate Array), or any other electronic device, system or arrangement. Accordingly, whilst the term server is used, this is for the purpose of example only and is not intended to be limiting.

Whilst the server 1200 is a shown as a single entity, it will be appreciated that the server 1200 can be distributed over a number of geographically separate locations, for example by using processing systems and/or databases 1201 that are provided as part of a cloud based environment. Thus, the above described arrangement is not essential and other suitable configurations could be used.

**Claims**

1. A computer-implemented method of identifying one or more candidate regions of one or more source proteins that are predicted to instigate an adaptive immunogenic response across a plurality of human leukocyte antigen, HLA, types, wherein the one or more source proteins has an amino acid sequence, the method comprising:

   (a) accessing the amino acid sequence of the one or more source proteins;
   (b) accessing a set of HLA types;
   (c) predicting an immunogenic potential of a plurality of candidate epitopes within the amino acid sequence, for each of the set of HLA types;
   (d) dividing the amino acid sequence into a plurality of amino acid sub-sequences;
   (e) for each of the plurality of amino acid sub-sequences, generating a region metric that is indicative of a predicted ability of the amino acid sub-sequence to instigate an immunogenic response across the set of HLA types, wherein the region metrics are based on the predicted immunogenic potentials of the plurality of candidate epitopes, for each of the set of HLA types; and
   (f) applying a statistical model to identify whether any of the generated region metrics are statistically significant, whereby an amino acid sub-sequence identified as having a statistically significant region metric corresponds to a candidate region of the amino acid sequence that is predicted to instigate an immunogenic response across at least a subset of the set of HLA types.

2. The computer-implemented method of claim 1, further comprising the step of assigning, for each of the set of HLA types, an epitope score to each amino acid, wherein the epitope score is based on the predicted immunogenic potentials of one or more of the candidate epitopes comprising that amino acid, for that HLA type; and wherein each of the region metrics is generated based on the epitope scores for the amino acids within the respective amino acid sub-sequence, across the set of HLA types.

3. The computer-implemented method of claim 1 or claim 2, wherein at least a subset of the epitope scores are assigned by:

(i) identifying a first plurality of candidate epitopes having a first length, across the amino acid sequence;
(ii) generating, for each of the set of HLA types, an epitope score for each of the first plurality of candidate epitopes that is indicative of the predicted immunogenic potential of the respective candidate epitope for that HLA type;
(iii) identifying a second plurality of candidate epitopes having a second length, across the amino acid sequence;
(iv) generating, for each of the set of HLA types, an epitope score for each of the second plurality of candidate epitopes that is indicative of the predicted immunogenic potential of the respective candidate epitope for that HLA type; and
(v) for each of the set of HLA types, assigning, for each amino acid of the amino acid sequence, the epitope score of the candidate epitope that is predicted to have the best immunogenic potential of all of the first and second candidate epitopes comprising that amino acid, for that HLA type.

4. The computer-implemented method of any of the preceding claims, wherein the candidate epitopes have a length of at least 8 amino acids, preferably wherein the candidate epitopes have a length of 8, 9, 10, 11, 12 or 15 amino acids.

5. The computer-implemented method of any of the preceding claims, wherein the predicted immunogenic potential of a candidate epitope for a particular HLA type is based on one or more of a predicted binding affinity and a predicted processing of the identified candidate epitope.

6. The computer-implemented method of any of the preceding claims, wherein the immunogenic potential of a candidate epitope is further based on a similarity of the candidate epitope to a human protein.

7. The computer-implemented method of any of claims 2 to 6, further comprising digitising the assigned epitope scores, wherein each epitope score meeting a predetermined criterion is transformed to a "1" and each epitope score not meeting the predetermined criterion is transformed to a "0".

8. The computer-implemented method of any of the preceding claims, wherein the set of HLA types includes HLA types of Major Histocompatibility Complex, MHC, Class I and HLA types of MHC Class II.

9. The computer-implemented method of any of the preceding claims, wherein the set of HLA types comprises HLA types representative of at least one human population group, preferably where the set of HLA types is representative of the human population.

10. The computer-implemented method of any of the preceding claims, wherein the set of HLA types comprises the top N most frequent HLA types within the human population or a human population group, preferably wherein N is at least 5, more preferably at least 50 and even more preferably at least 100.

11. The computer-implemented method of any of claims 1 to 8, wherein the set of HLA types is representative of a given individual.

12. The computer-implemented method of any of the preceding claims, wherein applying the statistical model comprises applying a Monte Carlo simulation to estimate a p-value for each of the generated region metrics.

13. The computer-implemented method of claim 12 when dependent on at least claim 2, wherein applying the Monte Carlo simulation includes:

(i) for each HLA type, arranging the epitope scores into a plurality of epitope segments and epitope gaps based on the distribution of the epitope scores; and
(ii) for each HLA type, iteratively generating a random arrangement of the epitope segments and epitope gaps.

14. The computer-implemented method of any of the preceding claims, further comprising applying a false discovery rate, FDR, procedure to the results of the statistical model, preferably wherein the FDR procedure is a Benjamini-Hochberg or Benjamini- Yekutieli procedure.

15. The computer-implemented method of any of claims 2 to 14, further comprising weighting the epitope scores dependent upon the human population frequency of the respective HLA type within the set of HLA types.

16. The computer-implemented method of any of the preceding claims, wherein each amino acid sub-sequence comprises at least 8 amino acids, preferably between 20 and 50 amino acids, more preferably between 50 and 150 amino acids.

17. The computer-implemented method of any of the preceding claims, wherein each of the region metrics is further indicative of a predicted B-cell response potential of the respective amino acid sub-sequence.

18. The computer-implemented method of claim 17 when dependent on claim 2, wherein each assigned epitope score is further based on the predicted B cell response potential of the respective amino acid.

19. The computer-implemented method of any of the preceding claims, further comprising analysing each candidate region of the one or more source proteins for the presence of B cell epitopes.

20. The computer-implemented method of any of the preceding claims, further comprising comparing each identified candidate region with at least one human protein sequence in order to determine a degree of similarity, and ranking or discarding the candidate regions based on the degree of similarity with at least one of the human proteins being greater than a predetermined threshold.

21. The computer-implemented method of any of the preceding claims, further comprising adjusting a candidate region based on one or more adjacent amino acid sub-sequences.

22. The computer-implemented method of any of the preceding claims, wherein the one or more source proteins are one or more proteins of a virus, tumour, bacterium or parasite, or fragments thereof, including neoantigens.

23. The computer-implemented method of any of the preceding claims, wherein the one or more source proteins are one or more proteins of a coronavirus, preferably the SARS-CoV-2 virus.

24. The computer-implemented method of any of the preceding claims, wherein the one or more source proteins comprise a plurality of variations of one or more proteins.

25. The computer-implemented method of claim 24, further comprising filtering the one or more candidate regions so as to select one or more candidate regions in conserved areas.

26. A method of creating a vaccine, comprising:

identifying at least one candidate region of at least one source protein by a method according to any of the preceding claims; and
synthesising the at least one candidate region and/or at least one predicted epitope within the at least one candidate region, or encoding the at least one candidate region and/or at least one predicted epitope within the at least one candidate region, into a corresponding DNA or RNA sequence.

27. A system for identifying one or more candidate regions of one or more source proteins that are predicted to instigate an immunogenic response across a plurality of human leukocyte, HLA allele types, wherein the one or more source proteins has an amino acid sequence, the system comprising at least one processor in communication with at least one memory device, the at least one memory device having stored thereon instructions for causing the at least one processor to perform a method according to any of claims 1 to 25.

EP 3 901 954 A1

28. A computer readable medium having computer executable instructions stored thereon for implementing the method of any of claims 1 to 25.

16

Fig. 1A    Epitope map of the S-Protein across the most frequent HLA-A, HLA-B and HLA-DRB alleles in the human population. Data is transformed such that a positive results for CD8 relates to 0.7 or above, and 0.1 or below for Class II. Broad coverage for CD8 and CD4 is demonstrated with overlaying B cell antibody support

Fig. 1A (Cont.)

## Fig. 1B

Wuhan - Antigen Presentation - S

T Cell class I

T Cell class II

HLA-DRB1*01:02   HLA-DRB1*01:01
HLA-DRB1*04:01   HLA-DRB1*03:01
HLA-DRB1*04:03   HLA-DRB1*04:02
HLA-DRB1*04:05   HLA-DRB1*04:04
HLA-DRB1*04:11   HLA-DRB1*04:07
HLA-DRB1*08:01   HLA-DRB1*07:01
HLA-DRB1*08:03   HLA-DRB1*08:02
HLA-DRB1*09:01   HLA-DRB1*08:07
HLA-DRB1*11:01   HLA-DRB1*10:01
HLA-DRB1*11:04   HLA-DRB1*11:03
HLA-DRB1*12:02   HLA-DRB1*12:01
HLA-DRB1*13:02   HLA-DRB1*13:01
HLA-DRB1*14:01   HLA-DRB1*13:03
HLA-DRB1*14:08   HLA-DRB1*14:02
HLA-DRB1*15:01   HLA-DRB1*14:54
HLA-DRB1*15:03   HLA-DRB1*15:02
HLA-DRB1*16:02   HLA-DRB1*16:01
Class II mean

80
60
40
20

B Cell

B Cell

0.50
0.25

0   100   200   300   400   500   600   700   800   900   1000   1100   1200

Protein sequence offset

EP 3 901 954 A1

Fig. 2

P0DTC2 - Class I (cut-off=0.7)

Hierarchical clustering of binary transformation of the epitope maps. Illustrated here for Class I CD8 epitopes in HLA-A and HLA-B alleles. Predictions for Class I are greater than 0.7 are transformed to 1. Illustrated for the S-Protein here for demonstrated purposes.

Fig. 3  Epitope hotspots from the Monte Carlo analysis are captured across the entire viral proteome using filtering procedures for conserved and human self-peptides. The most abundant signal for hotspots is in the orf1ab polyprotein.

# Fig. 4

Change in Antigen Presentation score

The scatter plot shows the mutated AP score (Y-axis) against its wildtype AP score (X-axis) protein variant. Each blue dot is one of the 1122 protein variants. The orange line is a least square fit with slope = 0.72. Variants on the green dashed line of slope 1 would show no change in AP after the introduction of the Mutation

# Fig. 5

Application of the Monte Carlo epitope hotspot prediction method to 10 mutating virus sequences in different geographical locations. The hotspots for 10 mutated sequences compared to the Wuhan reference seqeuence are on the x- axis, the fequence the epitope hotspots. The frequences are shown for 3 different epitope hotspot bin lengths; 27 (left), 50 (centre) and 100 (right). It is clear that the epitope hotspots are robust across mutating sequences, while occasionally new epitope hotsptots emerge in some squences in different geographical locations.

27

Legend:
- Australia_NSW12
- Congo_158
- Denmark_SSI-104
- France_Valence
- Iceland_315
- Italy_UniMI03
- Netherlands_NoordBrabant
- New_Zealand
- USA_NY-NYUMC2
- USA_WA-UW260
- Wuhan_reference

GenBank ID, Bin size: (N, 27) (ORF3a, 27) (ORF6, 27) (S, 27) (ORF7a, 27) (orf1ab, 27) (ORF8a, 27) (M, 27)

# Fig. 5 (Cont.)

# Fig. 6

The scatter plots show the distribution of the hotspot conservation score (Y-axis) for proteins in the viral genome (X-axis). Each plot contains the conservation scores of the hotspots identified based on immune presentation (IP), using different bin sizes: 27 (left), 50 (centre), and 100 (right). A hotspot is represented by a filled coloured circle, while the median conservation score for a given protein is depicted by a hollow square. As a reference, upwards facing triangles show the minimum conservation score for that protein. Only hotspots with a conservation score above the median were taken for further consideration.

Hotspot conservation scores per protein - bin size : 27

# Fig. 6 (Cont.)

Hotspot conservation scores per protein - bin size : 50

Hotspot conservation scores per protein - bin size : 100

# Fig. 7

S201 → Access amino acid sequence of one more source proteins

S203 → Identify a plurality of candidate epitopes within the amino acid sequence

S205 → Predict an immune response potential for each of the candidate epitopes, for each of a set of HLA types

S207 → Assign an epitope score to each amino acid, for each HLA type, based on the overlapping candidate epitope having the best predicted immunogenic potential for the HLA type

S208 → Digitise epitope scores

S209 → Divide the amino acid sequence into a plurality of amino acid sub-sequrnces

S211 → For each amino acid sub-sequence, calculate a region metric based on the assigned epitope scores

S213 → Apply a statistical model to identify candidate regions having statistically significant region metrics

S215 → Filter candidate regions occurring in conserved regions

# Fig. 8

# Fig. 9

Europäisches
Patentamt

European
Patent Office

Office européen
des brevets

## EUROPEAN SEARCH REPORT

Application Number

EP 20 17 0484

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/330335 A1 (BREMEL ROBERT D [US] ET AL) 12 December 2013 (2013-12-12) * paragraphs [0172] - [0228], [0260] - [0269], [0270] - [0282], [0296] - [0301], [0395]; claims 1-97 * | 1-15 | INV. G16B15/30 A61K39/00 G16B30/00 |
| A | MORTEN NIELSEN ET AL: "NetMHCpan-3.0; improved prediction of binding to MHC class I molecules integrating information from multiple receptor and peptide length datasets", GENOME MEDICINE, vol. 8, no. 1, 30 March 2016 (2016-03-30), XP055571478, DOI: 10.1186/s13073-016-0288-x * the whole document * | 1-15 | |
| A | GULUKOTA K ET AL: "HLA allele selection for designing peptide vaccines", GENETIC ANALYSIS: BIOMOLECULAR ENGINEERING, ELSEVIER SCIENCE PUBLISHING, US, vol. 13, no. 3, 19 March 1999 (1999-03-19) , pages 81-86, XP004070187, ISSN: 1050-3862, DOI: 10.1016/1050-3862(95)00156-5 * the whole document * | 1-15 | TECHNICAL FIELDS SEARCHED (IPC) G16B A61K G16H |
| A | TOMER HERTZ ET AL: "HIV-1 Vaccine-Induced T-Cell Reponses Cluster in Epitope Hotspots that Differ from Those Induced in Natural Infection with HIV-1", PLOS PATHOGENS, vol. 9, no. 6, 20 June 2013 (2013-06-20), page e1003404, XP055766261, DOI: 10.1371/journal.ppat.1003404 * the whole document * | 1-15 | |

-/--

The present search report has been drawn up for all claims

| Place of search | Date of completion of the search | Examiner |
|---|---|---|
| Munich | 18 January 2021 | Heidrich, Alexander |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 1 of 2

Europäisches
Patentamt

European
Patent Office

Office européen
des brevets

## EUROPEAN SEARCH REPORT

Application Number

EP 20 17 0484

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | Brandon Malone ET AL: "Artificial intelligence predicts the immunogenic landscape of SARS-CoV-2 leading to universal blueprints for vaccine designs.", bioRxiv, 21 April 2020 (2020-04-21), XP055760223, DOI: 10.1101/2020.04.21.052084 Retrieved from the Internet: URL:https://www.biorxiv.org/content/10.1101/2020.04.21.052084v1.full.pdf [retrieved on 2021-01-15] * the whole document * | 1-15 | |

----

TECHNICAL FIELDS
SEARCHED      (IPC)

The present search report has been drawn up for all claims

| Place of search | Date of completion of the search | Examiner |
|---|---|---|
| Munich | 18 January 2021 | Heidrich, Alexander |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 2 of 2

## ANNEX TO THE EUROPEAN SEARCH REPORT
## ON EUROPEAN PATENT APPLICATION NO.

EP 20 17 0484

This annex lists the patent family members relating to the patent documents cited in the above-mentioned European search report.
The members are as contained in the European Patent Office EDP file on
The European Patent Office is in no way liable for these particulars which are merely given for the purpose of information.

18-01-2021

| Patent document cited in search report | Publication date | Patent family member(s) | Publication date |
|---|---|---|---|
| US 2013330335 A1 | 12-12-2013 | EP 2550529 A1<br>US 2013330335 A1<br>WO 2011119484 A1 | 30-01-2013<br>12-12-2013<br>29-09-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020070307 A
- WO 2017186959 A

### Non-patent literature cited in the description

- **YANG et al.** *Nature,* 2004, vol. 428 (6982), 561-4
- **CHANNAPPANAVAR et al.** *Immunol Res,* 2014, vol. 88 (19), 11034-44
- **YANG et al.** *Clin Immunol,* 2006, vol. 120 (2), 171-8
- **LIU et al.** *JCI Insight,* 2019, vol. 4 (4
- **TIRADO ; YOON.** *Viral Immunol,* 2003, vol. 16 (1), 69-86
- **WAN et al.** *J Virol,* 2020, vol. 94 (5
- **MARSH et al.** *Tissue Antigens,* 2010, vol. 75 (4), 291-455
- **SHU, Y. ; J. MCCAULEY.** GISAID: Global initiative on sharing all influenza data - from vision to reality. *Euro Surveill,* 2017, vol. 22 (13
- **SIEVERS, F. ; D.G. HIGGINS.** Clustal Omega for making accurate alignments of many protein sequences. *Protein Sci,* 2018, vol. 27 (1), 135-145
- **DHANDA, S.K. et al.** IEDB-AR: immune epitope database-analysis resource in 2019. *Nucleic Acids Res,* 2019, vol. 47 (W1), W502-W506
- **GRIFONI, A. et al.** A Sequence Homology and Bioinformatic Approach Can Predict Candidate Targets for Immune Responses to SARS-CoV-2. *Cell Host Microbe,* 2020
- **SHU, Y. ; J. MCCAULEY.** GISAID: Global initiative on sharing all influenza data - from vision to reality. *Euro Surveill,* 31 March 2020, vol. 22 (13
- **HADFIELD, J. et al.** Nextstrain: real-time tracking of pathogen evolution. *Bioinformatics,* 2018, vol. 34 (23), 4121-4123